# EUROPEAN PATENT APPLICATION

(11) **EP 1 917 932 A1**
(43) Date of publication of application: **07.05.2008**
(21) Application number: 07254280.6
(22) Date of filing: 29.10.2007
(51) Int. Cl.: A61F 2/16

(54) **Piggyback lenses**

(30) Priority: 30.10.2006 US 589482
(71) Applicant: Calhoun Vision Inc., Pasadena, CA 91107 (US)
(72) Inventor: Maloney, Robert K., Los Angeles, CA 90049 (US)
(74) Representative: Jackson, Richard Eric

(57) **Abstract**

The invention relates to a system for improving vision using a pair of intraocular lenses. A standard or light adjustable intraocular lens is combined with a light adjustable "piggyback" lens such that the "piggyback" lens corrects any vision deficiencies not addressed by the first lens.

## Description

### TECHNICAL FIELD

The invention relates to intraocular lenses. Specifically it relates to an intraocular lens that "piggybacks" onto an existing lens such as an intraocular lens ("IOL") to address refraction errors in the existing lens. The "piggyback" IOL comprises a lens whose optical properties can be manipulated by exposing the lens to an external energy source such as light.

### BACKGROUND OF THE INVENTION

Approximately two million cataract surgery procedures are performed n the United States annually. The procedure generally involves making an incision in the anterior lens capsule to remove the cataractous crystalline lens and implanting an intraocular lens in its place. The power of the implanted lens is selected (based upon pre-operative measurements of ocular length and corneal curvature) to enable the patient to see without additional corrective measures (e.g., glasses or contact lenses). Unfortunately, due to errors in measurement, and/or variable lens positioning and wound healing, about half of all patients undergoing this procedure will not enjoy optimal vision without correction after surgery. Brandser et al., Acta Ophthalmol Scand 75:162 - 165 (1997; Oshika et al., J Cataract Refract Surg 24:509 - 514 (1998). Because the power of prior art intraocular lenses generally cannot be adjusted once they have been implanted, the patient typically must choose between replacing the implanted lens with another lens of a different power, being resigned to the use of additional corrective lenses such as glasses or contact lenses or submitting to another corrective procedure such as LASIK. Since the benefits of the first and third option do not outweigh the risks, they are almost never done.

One method for overcoming this problem is described in U.S. Patent No. 6,450,642. The invention described therein comprises a novel IOL whose optical properties can be more updated post implantation without the need of further invasive surgery. This allows adjustments to be made to the lens to overcome the errors discussed above. While this type of lens overcomes the problem for patients receiving new lenses, there remains a need for a means of addressing optical errors in standard IOL's after implantation.

### BRIEF SUMMARY OF THE INVENTION

In the present invention a second IOL is implanted within the patient's eye to address optical error encountered with the first IOL or to address changes in the patient's eyesight. The second or piggyback lens is generally placed in front of the previously implanted IOL or an existing natural lens and is fabricated so as to correct any aberrations in the IOL or natural lens. The piggyback lens typically comprises a lens whose optical properties can be manipulated or adjusted post implantation. This is generally done by exposing at least a portion of the piggyback lens to an eternal energy source such as light.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

FIG. 1 is a cross-section of an eye showing the placement of the piggyback lens with the capsular bag.

FIG. 2 is a cross-section of an eye showing placement of the piggyback lens in the sulcus between the capsular bag and the iris.

FIG. 3 is a cross-section of an eye showing the placement of the piggyback lens in the anterior chamber.

### DETAILED DESCRIPTION OF THE INVENTION

The invention relates to a piggyback IOL designed to work with a previously implanted IOL or with the patient's natural lens to improve the eyesight of the patient. The lens is generally placed anterior to the previously implanted IOL or natural lens and corrects any refractive errors that may exist. In the discussion that follows, the piggyback IOL is used in conjunction with a previously implanted IOL. Those skilled in the art will recognize that the principles are readily applied to situations where the patient's natural lens is still in place.

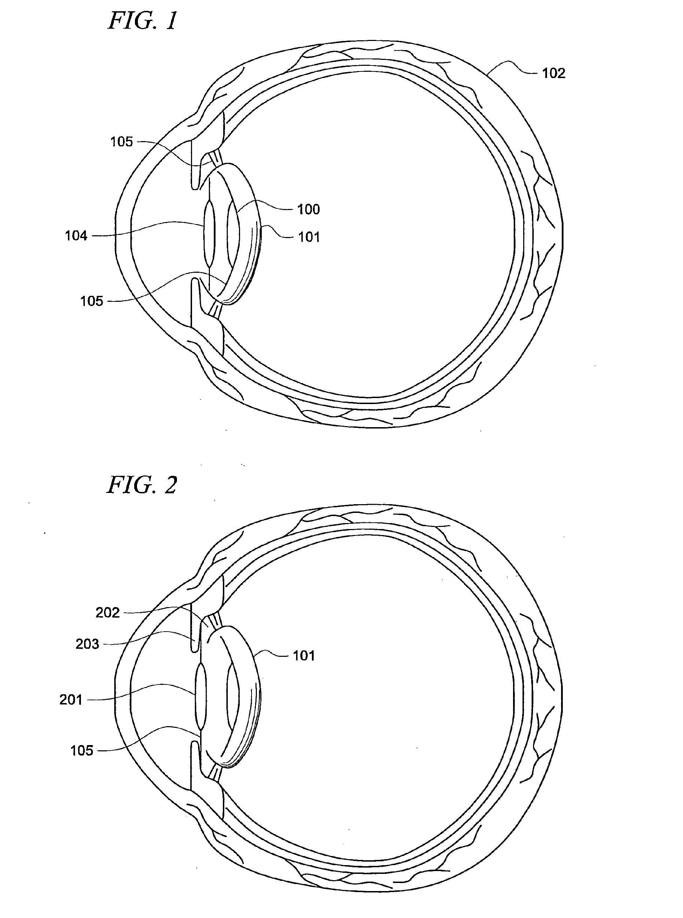

Referring to Fig. 1, a previously implanted IOL 100 is shown in the capsular bag 101 of the patient's eye 102. In this embodiment, the piggyback IOL 104 is placed within the capsular bag 101 just in front of the previously implanted IOL 100.
The piggyback lens 104 is inserted using standard surgical techniques and is anchored to the capsular bag using conventional anchoring means such as haptics 105.

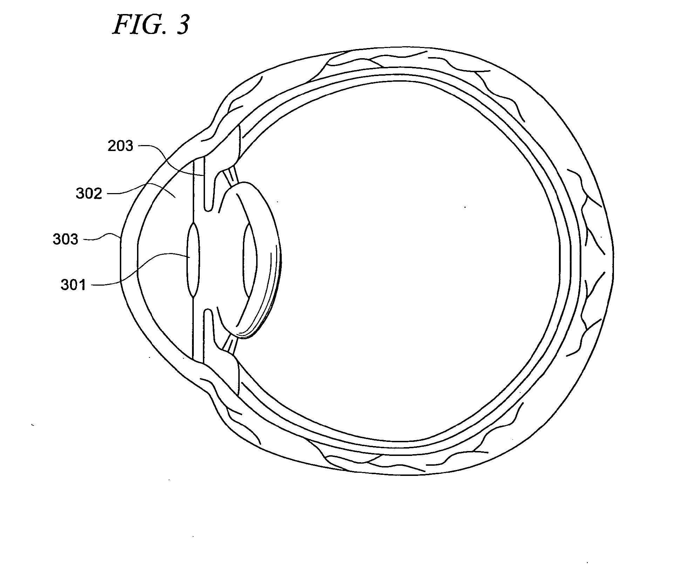

In another embodiment, shown in Fig. 2, the piggyback IOL 201 is placed in the sulcus 202 between the iris 203 and the capsular bag 101. Again, the piggyback IOL is implanted using standard surgical techniques and is anchored using conventional anchoring means such as haptics 105.

In still another embodiment, the piggyback IOL 301 is placed in the anterior chamber 302 between the iris 203 and the cornea 303.

The piggyback IOL is an adjustable IOL such as those described in U.S. Patent No. 6,450,642, the teachings of which are hereby incorporated by reference for purposes of United States Patent practice.

The piggyback lenses which comprise adjustable lenses whose optical properties can be manipulated after implantation. One example of these lenses is described in U.S. Patent No. 6,450,642. In this embodiment, the piggyback lens comprises a lens having macromers dispersed throughout the lens. The macromers are capable of energy induced polymerization such as photopolymerization. When at least a portion of the lens is exposed to an external radiation source, the macromers in the region exposed to the radiation polymerize, resulting in a change in the optical properties of the lens. This can be the result of changes in the shape of the lens, changes in the optical properties of the lens or both. Typically, the energy used is light with UV light preferred.

Where an adjustable IOL is used, the basic lens may have zero power. This is achieved by either using a plate lens with both surfaces having zero power or a curved lens where the power of the front surface combines with the power of the rear surface equal zero. Alternatively, the lens may have some corrective pouch which can be adjusted after implantation using the method described above.

The piggyback IOL of the invention can be found in several different shapes including meniscus, biconvex and flat plate. The shape of the lens may depend upon where the lens is to be placed. For example, where the piggyback lens is to be inserted into the capsular bag along with a pre-existing IOL, a meniscus shaped lens may be used to help ensure that the two lenses do not touch. Should the lenses touch, there is the possibility that this will cause cell growth between the two lenses which in turn could lead to secondary cataracts or Posterior Cavity Opacity ("PCO").

The following procedure is used to implant an adjustable lens, the piggyback lens. The process begins by determining the patient's optical requirements 401. Once the requirements have been determined, a lens blank is selected and inserted into the patient's eye 402. Once the lens has been inserted and wound healing has occurred, at least a portion of the lens is exposed to an external radiation source 403 such that the macromers present are induced to polymerize. The regions exposed to the entry source and the duration of the exposure will depend on at least in part on the desired optical to be achieved. For example, where multifocality is desired, a multifocal pattern of exposure such as that disclosed in published U.S. Patent Application 20050187622 may be employed.

After the initial exposure has occurred and the changes in the optical properties stabilizes, the patient is then re-examined to determine if the desired optical properties have been achieved 404. If the properties have been achieved, then the entire lens may be exposed to an energy source to lock-in the optical properties achieved 405. In the case of re-adjustable light adjustable lenses ("LALs"), such as those described in U.S. Patent No. 6,851,804, this lock-in step is not required.

If the desired optical properties were not achieved after the initial exposure, steps 403 and 404 may be repeated until the desired properties are achieved.

The piggyback lenses can be used to correct vision problems of patients' natural lenses as well as IOLs. This would be especially true in extreme cases such as myopia, hypeopia and/or astigmatism. In these cases, the piggyback IOL is again inserted anterior to the natural lens, preferably in the sulcus or in the anterior chamber, much like the positions shown in Figs. 2 and 3

In yet another embodiment, the adjustable piggyback lens is implanted at the same time as a standard IOL is implanted. In this embodiment, the piggyback lens allows correction of vision problems that may occur during healing as well as those that were undetected before implantation of the lens. When a re-adjustable lens is used, such as that described in U.S. Patent No. 6,851,804, the embodiment also allows for correction of vision problems that may develop during the life of the patient.

In this embodiment, the patient's natural lens is removed using procedures known in the art such as phaeco-emulsification. A conventional intraocular lens is then implanted, typically with the capsular bag. An adjustable piggyback lens is then implanted anterior to the conventional IOL. The lenses combined will typically have optical properties such that they meet the requirements determined prior to removal of the natural lens. As described above, the piggyback lens may be inserted into the capsular bag, into the sulcus between the iris and the capsular bag or into the anterior chamber.

After both lenses have been placed, the patient is examined to determine if the dual lens system possess the optical properties to meet the patient's need. If no further correction is needed the power of the piggyback lens may be "locked-in" when conventional adjustable lenses are used or may be left unaltered in the case of a re-adjustable lens.

Where further correction is required, at least a portion of the piggyback lens is exposed to an external energy source in the manner described above. When the desired correction has been achieved, the properties of the piggyback lens may be "locked-in" in the manner described above.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. An optical system comprising:
an intraocular lens;
an adjustable piggyback intraocular lens;
placed such that said piggyback intraocular lens corrects optical irregularities of said intraocular lens.

2. An optical system comprising:
a natural lens;
an adjustable piggyback lens positioned such that it corrects optical irregularities
in
said natural lens.

3. The system of claim 1 or claim 2 wherein the optical properties of said piggyback lens can be manipulated by exposing at least a portion of said piggyback lens to an external energy source.

4. The system of claim 3 wherein said external energy source comprises light.

5. The system of claim 3 wherein said external energy source comprises ultraviolet light.

6. A method for correcting vision comprising:
determining the optical requirements of a patient;
inserting an adjustable piggyback lens anterior to a pre-existing lens in the patient's eye.

7. The method of claim 6 wherein the piggyback lens is inserted into the anterior chamber of the patient's eye.

8. The method of claim 6 wherein said pre-existing lens is an intraocular lens.

9. The method of claim 6 wherein the pre-existing lens is the patient's natural lens.

10. A method for correcting vision comprising:
removing a patient's natural lens;
inserting an intraocular lens with the patient's capsular bag;
inserting an adjustable piggyback lens anterior to the intraocular lens.

11. The method of claim 6 or claim 10 wherein the piggyback lens is inserted into the capsular bag of the eye.

12. The method of claim 6 or claim 10 wherein the piggyback lens is inserted into the sulcus between the iris and the capsular bag.

13. The method of claim 10 wherein the piggyback lens is inserted into the interior chamber of the eye.

14. The method of any one of claims 6 to 12 wherein the piggyback lens comprises a light adjustable lens.

15. A method for correcting vision comprising:
determining the optical requirements of a patient;
inserting a piggyback lens anterior to a pre-existing lens in the patient's eye wherein the optical properties of the piggyback lens can be manipulated by exposing said piggyback lens to an external energy source.

16. The method of any one of claims 6 to 15 further comprising the step of:
exposing a least a portion of said piggyback lens to an energy source to induce changes in the optical properties of said piggyback lens.

17. The method of claim 16 wherein said energy source is light.

18. The method of claim 16 wherein said energy source is ultraviolet light.

19. The method of claim 15 wherein said piggyback lens is placed in the capsular sac of said eye.

20. The method of wherein said piggyback lens is placed in the sulcus.

21. The method of claim 15 wherein said piggyback lens is placed in the anterior chamber.

22. The method of claim 15 wherein the pre-existing lens is an intraocular lens.

23. The method of claim 15 wherein the pre-existing lens is a natural lens.
